# EUROPÄISCHE PATENTANMELDUNG

(11) **EP 3 838 192 A1**
(43) Veröffentlichungstag der Anmeldung: **23.06.2021**
(21) Anmeldenummer: 19401053.4
(22) Anmeldetag: 16.12.2019
(51) Int. Cl.: A61B 17/3207, A61B 17/22, A61B 17/3203, A61B 17/00

(54) **KATHETEREINRICHTUNG MIT ELEKTRISCHER SPULE**

(71) Anmelder: BIOTRONIK AG, 8180 Bülach (CH)
(72) Erfinder: Fargahi, Amir, 8180 Bülach (CH)
(74) Vertreter: Randoll, Sören

(57) **Zusammenfassung**

Die Erfindung betrifft eine Kathetereinrichtung (1) geeignet zum Öffnen einer Okklusion (CTO) in einem Gefäß (G), mit einem entlang einer Längsachse (x) erstreckten Katheterschaft (2), der einen distalen Endabschnitt (2a) aufweist. Erfindungsgemäß ist vorgesehen, dass die Kathetereinrichtung (1) ein ferromagnetisches Projektil (12) aufweist sowie eine am distalen Endabschnitt (2a) des Katheterschafts (2) angeordnete elektrische Spule (3) zum Beschleunigen des Projektils (12).

## Beschreibung

Die Erfindung betrifft eine Kathetereinrichtung geeignet zum Öffnen einer Okklusion (CTO) in einem Gefäß, mit einem entlang einer Längsachse erstreckten Katheterschaft, der einen distalen Endabschnitt aufweist. Erfindungsgemäß ist vorgesehen, dass die Kathetereinrichtung ein ferromagnetisches Projektil aufweist sowie eine am distalen Endabschnitt des Katheterschafts angeordnete elektrische Spule zum Beschleunigen des Projektils.

Derartige Kathetereinrichtungen können beispielsweise zum Rekanalisieren bzw. Wiedereröffnen eines chronischen, im Wesentlichen vollständigen Verschlusses (Chronic Total Occlusion oder kurz CTO) eines Gefäßes eines Patienten verwendet werden. Andere Anwendungen wie hierin beschrieben, können ebenfalls mit der hierin vorgestellten Kathetereinrichtung getätig werden.

Derartige CTOs kommen bei ca. 20% aller Patienten im Herzkatheter-Labor vor. Die durchschnittliche Interventionsdauer liegt in der Regel bei etwa zwei bis 2,5 Stunden.

Zur Behandlung bzw. Rekanalisierung von chronischen Totalverschlüssen (CTOs) sind aus dem Stand der Technik Einrichtungen bekannt, die auf manueller Rotation eines Drahtes zur Rekanalisierung der Okklusion basieren. Derartige Prozesse können jedoch sehr zeitaufwendig und daher weniger effizient sein.

Hiervon ausgehend liegt der vorliegenden Erfindung daher die Aufgabe zugrunde, eine Kathetereinrichtung bereitzustellen, die hinsichtlich der oben geschilderten Problematik verbessert ist.

Diese Aufgabe wird durch eine Kathetereinrichtung mit den Merkmalen des Anspruchs 1 gelöst.

Vorteilhafte Ausgestaltungen sind in den entsprechenden Unteransprüchen angegeben und werden nachfolgend beschrieben.

Demgemäß wird eine Kathetereinrichtung beispielsweise geeignet zum Rekanalisieren einer Okklusion (insbesondere CTO) in einem Gefäß offenbart, mit:
- einem entlang einer Längsachse erstreckten Katheterschaft, der einen distalen Endabschnitt aufweist.

Erfindungsgemäß ist vorgesehen, dass die Kathetereinrichtung ein vorzugsweise ferromagnetisches Projektil aufweist sowie eine am distalen Endabschnitt angeordnete elektrische Spule zum Beschleunigen des Projektils durch Erzeugung eines Magnetfeldes mittels der Spule.

Das ferromagnetische Projektil muss dabei nicht ausschließlich aus einem ferromagnetischen Material gefertigt sein. Es ist ausreichend, dass der Anteil des ferromagnetischen Materials derart gewählt ist, dass das Projektil mit der Spule zum Aufbrechen der Okklusion beschleunigbar ist.

Das Projektil und die elektrische Spule bilden also mit anderen Worten Komponenten einer ferromagnetischen Gaußkanone.

Der Katheterschaft der Kathetereinrichtung ist vorzugsweise flexibel ausgebildet, so dass dieser beim Einführen in das Gefäß in ausreichender Weise Krümmungen des Gefäßes folgen kann.

Der Katheterschaft weist des Weiteren vorzugsweise ein durchgängiges Lumen auf, das sich von einem proximalen Ende des Katheterschafts zu einem distalen Ende des Katheterschafts erstreckt.

Gemäß einer Ausführungsform der Erfindung ist vorgesehen, dass die elektrische Spule das Lumen in einer Umfangsrichtung des Katheterschafts am distalen Endabschnitt des Katheterschafts umgibt.

Im Rahmen der vorliegenden Erfindung bezieht sich der Begriff distal auf einen Teil oder eine Komponente des Systems, der bzw. die entlang des Katheterschafts von einem Griff der Kathetereinrichtung oder von einem Arzt, der die Kathetereinrichtung bedient, weiter entfernt ist, als ein entsprechender proximaler Teil bzw. eine entsprechende proximale Komponente der Kathetereinrichtung, der bzw. die entlang des Katheterschafts vergleichsweise näher am Griff oder näher am Arzt gelegen ist.

Gemäß einer Ausführungsform ist die Spule vorzugsweise an dem distalen Endabschnitt des Katheterschafts festgelegt. Insbesondere kann die Spule in eine Wandung des Katheters eingebettet sein oder kann zwischen zwei Materiallagen einer Wandung des Katheterschafts angeordnet sein.

Weiterhin ist gemäß einer Ausführungsform der Erfindung vorgesehen, dass das Projektil über ein Federelement mit einem in dem Lumen angeordneten oder in das Lumen einführbaren längserstreckten Element verbunden ist. Insbesondere ist das längserstreckte Element an der Kathetereinrichtung festgelegt oder festlegbar. Gemäß einer Ausfiihrungsform ist vorgesehen, dass das längserstreckte Element dazu ausgebildet ist, an einem proximalen Endabschnitt des Katheterschafts festgelegt zu werden. Mittels des längserstreckten Elements kann eine Bewegung des Projektils in distaler Richtung begrenzt werden. Dies ist bevorzugt, da vermieden werden muss, dass das Projektil im Körper des Patienten verloren geht. Weiterhin ermöglicht das längserstreckte Element das Vorschieben des Projektils im Lumen des Katheterschafts zur Okklusion hin sowie insbesondere ein genaues Positionieren des Projektils bezüglich der Spule. Das längserstreckte Element weist dabei vorzugsweise eine definierte Länge auf, derart, dass das damit verbundene Projektil an der richtigen Position bezüglich der Spule zu liegen kommt, wenn das längserstreckte Element bestimmungsgemäß in dem Lumen des Katheterschafts angeordnet ist.

Gemäß einer Ausführungsform der Erfindung ist das längserstreckte Element durch einen Metalldraht gebildet. Anstelle eines Metalldrahtes ist auch ein anderes längserstrecktes Element denkbar, das nicht biegeschlaff ist.

Gemäß einer Ausführungsform der Erfindung ist das längserstreckte Element integral mit dem Federelement ausgebildet. So können das längserstreckte Element und das Federelement z.B. aus einem Stück gefertigt sein.

Weiterhin ist gemäß einer Ausführungsform der Erfindung vorgesehen, dass das Federelement aus einer diamagnetischen Stahllegierung besteht und/oder dass das längserstreckte Element aus einer diamagnetischen Stahllegierung besteht. Hierdurch werden die Auswirkungen der Spule bzw. der Gaußkanone auf das längserstreckte Element bzw. auf das Federelement mit Vorteil reduziert.

Weiterhin ist gemäß einer Ausführungsform der Erfindung vorgesehen, dass das Projektil aus einer Anfangsposition, in der das Projektil in einem distalen Endabschnitt des Lumens angeordnet ist, durch Erzeugen eines Magnetfeldes mittels der elektrischen Spule in eine Endposition beschleunigbar ist, in der das Projektil durch eine distale Öffnung des Katheterschafts aus dem Katheterschaft (bzw. dem Lumen) heraussteht und dabei gegen die Okklusion stößt um diese mechanisch zu Öffnen.

Gemäß einer Ausfiihrungsform der Erfindung ist das Federelement dazu konfiguriert, durch Bewegen des Projektils in die Endposition gespannt zu werden, so dass das Federelement eine rückstellende Kraft auf das Projektil ausübt, die das Projektil zurück in die Anfangsposition bewegt. In dieser Ausführungsform kann die Kathetereinrichtung zur schnellen wiederholten Bearbeitung der Okklusion verwendet werden, um eine schnelle Öffnung zu erreichen.

Weiterhin ist gemäß einer bevorzugten Ausführungsform vorgesehen, dass die Kathetereinrichtung eine Steuereinheit aufweist, die dazu konfiguriert ist, einen die elektrische Spule durchfließende Strom so zu steuern, dass das Magnetfeld wiederholt an und ausgeschaltet wird, derart, dass das Projektil wiederholt zwischen der Anfangsposition und der Endposition hin- und her bewegt wird.

Gemäß einer Ausführungsform ist die Steuereinheit mittels entlang des Katheterschafts geführten elektrischen Leitern mit der elektrischen Spule verbunden, um die elektrische Spule zur Erzeugung des Magnetfeldes mit elektrischer Energie bzw. dem steuerbaren elektrischen Strom zu versorgen.

Gemäß einer weiteren Ausführungsform der Erfindung ist vorgesehen, dass die Kathetereinrichtung einen Führungsdraht aufweist, der in das Lumen einführbar ist, so dass der Katheterschaft entlang des Führungsdrahtes zur Okklusion des Gefäßes führbar ist.

Gemäß einer weiteren Ausführungsform der Erfindung ist vorgesehen, dass das Lumen des Katheterschafts einen Innendurchmesser im Bereich von 0,35 mm bis 0,45 mm aufweist, wobei der Innendurchmesser des Lumens insbesondere 0,4 mm beträgt.

Weiterhin ist gemäß einer Ausführungsform der Erfindung vorgesehen, dass das längserstreckte Element einen Durchmesser im Bereich von 0,15 mm bis 0,25 mm aufweist, wobei der Durchmesser des längserstreckten Elements insbesondere 0,2 mm beträgt.

Weiterhin kann das Federelement gemäß einer Ausführungsform der Erfindung einen Durchmesser im Bereich von 0,3 mm bis 0,4mm aufweisen, wobei der Durchmesser insbesondere 0,4 mm beträgt. Der Durchmesser des Federelements ist dabei kleiner als der Innendurchmesser des Lumens.

Ein weiterer Aspekt der vorliegenden Erfindung betrifft ein Verfahren zum Öffnen einer Okklusion eines Gefäßes, wobei das Verfahren eine erfindungsgemäße Kathetereinrichtung verwendet. Das Verfahren weist dabei die Schritte auf:
- Einführen eines Führungsdrahtes in das Gefäß,
- Einführen des Katheterschafts in das Gefäß, wobei der Führungsdraht im Lumen des Katheterschafts angeordnet wird und der Katheterschaft entlang des Führungsdrahtes zur Okklusion geführt wird,
- Entfernen des Führungsdrahts aus dem Lumen und Einführen des Projektils in das Lumen des Katheterschafts zusammen mit dem längserstreckten Element und dem Federelement, und
- wiederholtes Beschleunigen des Projektils gegen die Okklusion zum Öffnen der Okklusion.

Gemäß einer Ausführungsform weist das Verfahren den weiteren Schritt auf:
- Entfernen des längserstreckten Elementes, des Federelements und des Projektils aus dem Lumen des Katheterschafts.

Weiterhin weist das Verfahren gemäß einer Ausführungsform den weiteren Schritt auf:
- Einführen eines Führungsdrahts in die geöffnete Okklusion über das Lumen des Katheterschafts.

Schließlich weist das Verfahren gemäß einer Ausführungsform den weiteren Schritt auf:
- Entfernen des Katheterschafts aus dem Gefäß.

Mit dem nunmehr in der Okklusion platzierten Führungsdraht können anschließend ggf. weitere Maßnahmen (z.B. eine Angioplastie) durchgeführt werden.

Des Weiteren können mit der hierin vorgestellten Kathetereinrichtung auch Körperlumina wieder geöffnet oder geschaffen werden, die nicht unter das Krankheitsbildes einer CTO fallen. So ist angedacht auch Gefäßverschlüsse im peripheren Bereich mit der hierin vorgestellten Kathetereinrichtung zu durchstoßen. Ferner können auch subkutan Kanäle erschlossen werden um beispielsweise kleine Implantate wie Elektroden zu setzen oder Wirkstoffe gezielt einzubringen. So hingehend kann das hierin vorgestellte Verfahren mittels der hierin vorgestellten Kathetereinrichtung auch auf diese weiteren Indikationen angewendet werden.

In diesem Zusammenhang ist es vorgesehen, dass die hierin vorgestellte Kathetereinrichtung in automatisierter Form verwendet werden kann. Dies kann durch eine oszillierende Pulsation ermöglicht werden, welche auch programmierte werden kann. In einer bevorzugten Ausführungsform kann so eine geeignete Programmabfolge eingestellt werden, die eine individualisierte und für eine spezifische Indikation und Anwendung optimierte Therapie ermöglicht. In einer solchen Ausführungsform kann die hierin vorgeschlagene Kathetereinrichtung optimiert zum auto-dottering eingesetzt werden.

Im Folgenden sollen Ausführungsformen sowie weitere Merkmale und Vorteile der Erfindung anhand der Figuren erläutert werden. Es zeigen:
- Fig. 1: eine schematische Schnittansicht eines Katheterschafts einer Ausführungsform einer erfindungsgemäßen Kathetereinrichtung, wobei an eine distalen Endabschnitt des Katheterschafts eine mit elektrischer Spule vorgesehen ist;
- Fig. 2: eine schematische Schnittansicht eines Projektils der Kathetereinrichtung;
- Fig. 3: eine schematische Schnittansicht der Kathetereinrichtung, wobei das Projektil in seiner Anfangsposition angeordnet ist;
- Fig. 4: eine schematische Schnittansicht der Kathetereinrichtung, wobei das Projektil mittels der Spule in Richtung auf eine Endposition beschleunigt wird;
- Fig. 5: eine schematische Schnittdarstellung eines Gefäßes mit einer Okklusion, die geöffnet werden soll, wobei ein Führungsdraht in das Gefäß eingeführt ist, um den Katheterschaft einer erfindungsgemäßen Kathetereinrichtung zur Okklusion zu führen;
- Fig. 6: eine schematische Schnittdarstellung des Gefäßes gemäß Figur 5, wobei der Katheterschaft über den Führungsdraht zur Okklusion geführt wurde;
- Fig. 7: eine schematische Schnittdarstellung des Gefäßes gemäß Figur 6, wobei der Führungsdraht entfernt worden ist und ein Projektil der Kathetereinrichtung in das Lumen des Katheterschafts eingeführt ist;
- Fig. 8: eine schematische Schnittdarstellung des Gefäßes gemäß Figur 7, wobei die Okklusion mittels des Projektils geöffnet wird, in dem dieses mittels der Spule gegen die Okklusion beschleunigt wird und dabei mechanisch auf die Okklusion einwirkt;
- Fig. 9: eine schematische Schnittdarstellung des Gefäßes gemäß Figur 8 mit geöffneter Okklusion, wobei das Projektil aus dem Lumen des Katheterschafts entfernt worden ist;
- Fig. 10: eine schematische Schnittdarstellung des Gefäßes gemäß Figur 9, wobei ein Führungsdraht über das Lumen des Katheterschafts in die geöffnete Okklusion eingeführt ist; und
- Fig. 11: eine schematische Schnittdarstellung des Gefäßes gemäß Figur 10, wobei der Katheterschaft aus dem Gefäß entfernt worden ist und der in die Okklusion eingeführte Führungsdraht in der Okklusion verbleibt um ggf. eine Angioplastie der Okklusion vorzunehmen.

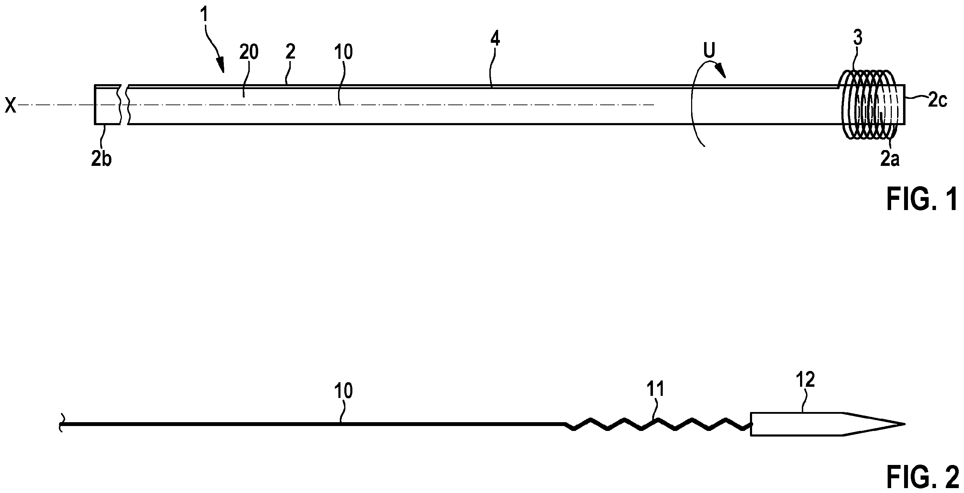

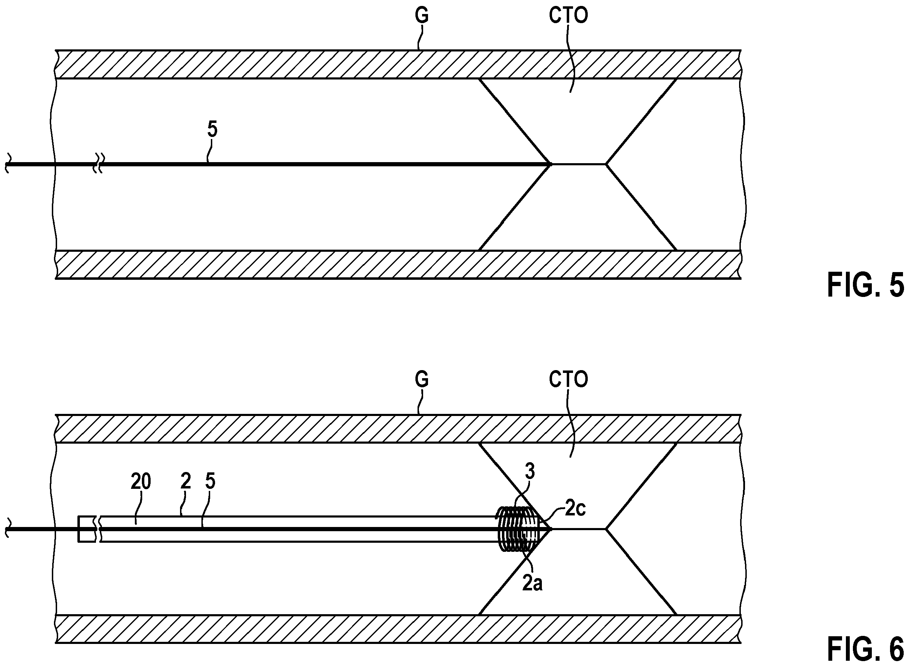

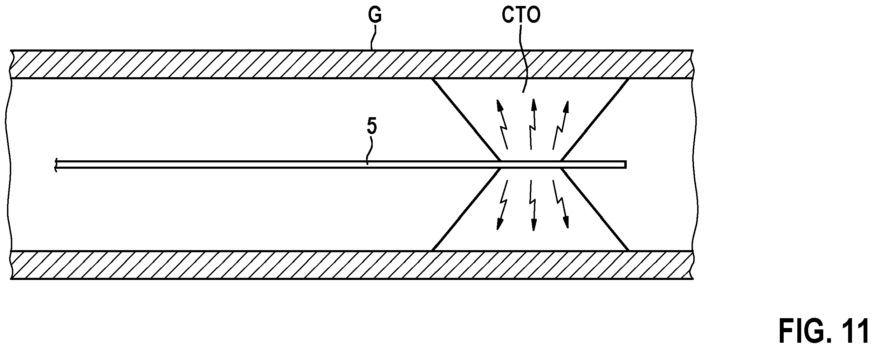

Die Figuren 1 bis 4 zeigen Ausführungsformen einer erfindungsgemäßen Kathetereinrichtung 1 zum Rekanalisieren einer Okklusion CTO in einem Gefäß G (vgl. Figuren 5 bis 11). Die Kathetereinrichtung 1 weist danach einen entlang einer Längsachse x erstreckten Katheterschaft 2 auf, der einen distalen Endabschnitt 2a aufweist. Erfindungsgemäß ist dabei vorgesehen, dass die Kathetereinrichtung 1 zum Rekanalisieren bzw. Öffnen der Okklusion CTO ein ferromagnetisches Projektil 12 aufweist sowie eine am distalen Endabschnitt 2a des Katheterschafts 2 angeordnete elektrische Spule 3 zum Beschleunigen des Projektils 12. Das Projektil 12 kann an einem distalen Ende des Projektils 12 eine Spitze ausbilden.

Ein Außendurchmesser des Katheterschafts 1 kann dem Außendurchmesser eines Standard- Führungskatheters entsprechen. Ein Innendurchmesser des Katheterschafts bzw. des Lumens 20 des Katheterschafts 2 kann z.B. 0,40mm betragen (kompatibel zu einem Standard-Führungsdraht 0.014").

Die elektrischen Zuleitungen 4 zur Spule 3 können z.B. auf einer Oberfläche des Katheterschafts 2 montiert sein. Ein Durchmesser eines für die Zuleitungen bzw. elektrischen Leiter 4 verwendeten isolierten Drahtes kann z.B. 25 µm betragen.

Eine Bewegung des Projektils 12 wird in distaler Richtung D durch ein längserstrecktes Element 10 bzw. einen Einführungsdraht 10 für das Projektil 12 begrenzt, der über ein Federelement 11 an dem Projektil 12 festgelegt ist.

Das längserstreckte Element (Einführungsdraht) 10 kann aus einer diamagnetischen Stahllegierung gefertigt sein und kann z.B. einen Durchmesser von 0,2 mm aufweisen. Das Federelement kann ebenfalls aus einer diamagnetischen Stahllegierung gefertigt sein (insbesondere aus der gleichen Stahllegierung wie das Element 10) und kann z.B. einen Durchmesser von 0,35 mm aufweisen.

Das Projektil 12 ist hingegen vorzugsweise aus einer ferromagnetischen Stahllegierung gefertigt oder weist zumindest einen ferromagnetischen Materialbereich auf. Weiterhin kann das Projektil 12 eine Beschichtung aufweisen. Das Projektil 12 kann z.B. einen Durchmesser von 0,4 mm und eine Länge (entlang der Längsachse x) von 5 mm aufweisen.

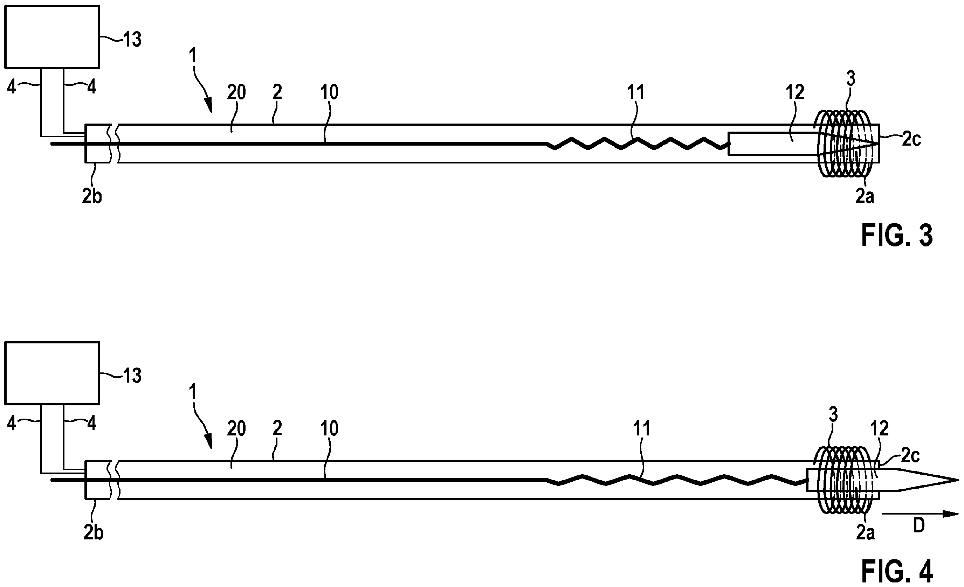

Die Spule 3 und das Projektil 12 stellen Komponenten einer ferromagnetischen Gaußkanone dar. Hierbei wird das ferromagnetische Projektil 12 aus einer Anfangsposition heraus mit Hilfe elektromagnetischer Kräfte beschleunigt, wobei ein entsprechendes Magnetfeld mittels der Spule 3 erzeugt wird. Hierzu wird durch die Spule 3 ein elektrischer Strom geleitet. Das dabei erzeugte Magnetfeld zieht das in der Anfangsposition bevorzugt vor der Spule 3 angeordnete Projektil an (vgl. Fig. 3) und beschleunigt es so in Richtung auf das Spulenzentrum, so dass sich das Projektil 12 in eine Endposition bewegt (vgl. Fig. 4). Das Magnetfeld wird dabei vorzugsweise abgeschaltet, bevor das Projektil 12 das Spulenzentrum (bezogen auf die Längsachse x) erreicht, um eine bremsende Wirkung zu vermeiden.

Das Federelement 11 ist dazu konfiguriert, durch Bewegen des Projektils 12 in distaler Richtung in die Endposition gespannt zu werden, so dass das Federelement 11 eine rückstellende Kraft auf das Projektil 12 ausübt, die das Projektil 12 (insbesondere bei ausgeschaltetem Magnetfeld) zurück in seine Anfangsposition bewegt, von der aus es wieder durch die Spule 3 bzw. deren Magnetfeld beschleunigbar ist.

Vorzugsweise weist die Kathetereinrichtung 1 diesbezüglich eine Steuereinheit 13 auf, die dazu konfiguriert ist, einen die elektrische Spule 3 durchfließenden Strom so zu steuern, dass das Magnetfeld wiederholt an und ausgeschaltet wird, derart, dass das Projektil 12 wiederholt zwischen der Anfangsposition und der Endposition hin- und her bewegt wird. Die Steuereinheit 13 kann gemäß Figuren 3 und 4 mittels der entlang des Katheterschafts 2 geführten elektrischen Leiter / Zuleitungen 4 mit der elektrischen Spule 3 verbunden sein. Das Projektil 12 kann somit periodisch gegen die Okklusion CTO stoßen und diese dabei durchgängig machen.

Die erfindungsgemäße Kathetereinrichtung 1 kann z.B. gemäß den Figuren 5 bis 11 in der folgenden Weise verwendet werden, um einen Führungsdraht 5 in der wiedereröffneten Okklusion CTO anzuordnen bzw. durch diese hindurchzuführen.

Hierbei kann die Okklusion CTO zunächst beurteilt werden, wobei sodann ein Führungsdraht 5 bis zur Okklusion CTO in das betreffende Gefäß G eingeführt wird (vgl. Fig. 5).

Sodann kann der Katheterschaft 2 über den Führungsdraht 5 bis zur Okklusion CTO in das Gefäß G eingerührt werden (vgl. Fig. 6).

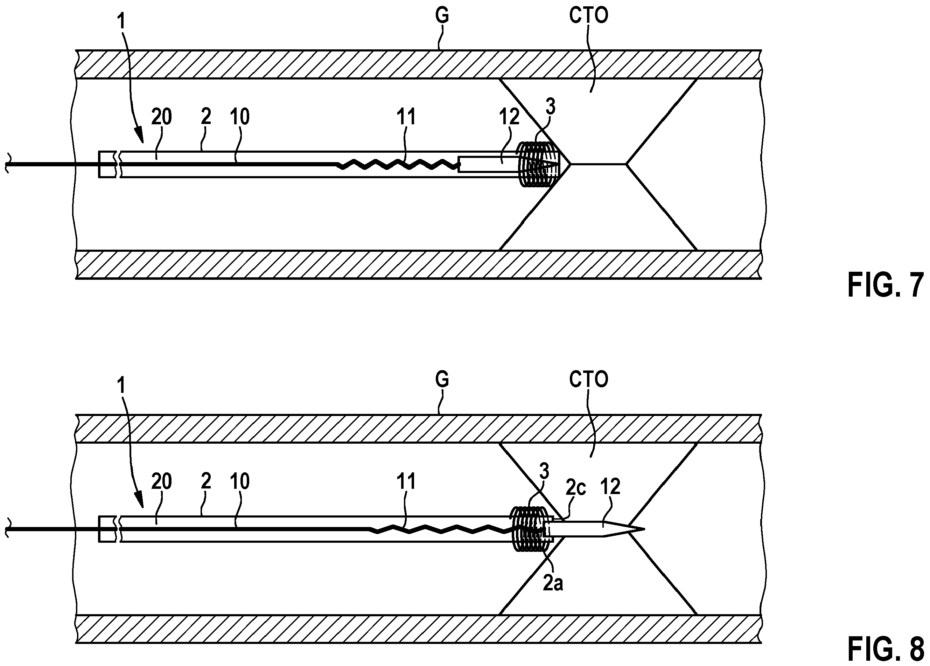

Der Führungsdraht 5 wird sodann entfernt und das Projektil 12 sowie das daran festgelegte Federelement 11 wird über das längserstreckte Element / Einführungsdraht 10 in das Lumen 20 des Katheterschafts 2 eingeführt. Das Element 10 wird am proximalen Endabschnitt 2b des Katheterschafts 2 fixiert (vgl. Fig. 7).

Die Gaußkanone stößt nun das Projektil 12 z.B. 2x pro Sekunde nach vorne in distaler Richtung zum Öffnen der Okklusion CTO. Das Federelement 11 zieht das Projektil 12 zurück. Die Amplitude des Projektils 12 wird vorzugsweise am proximalen Ende der Einrichtung 1 durch den Anwender eingestellt (vgl. Fig. 8).

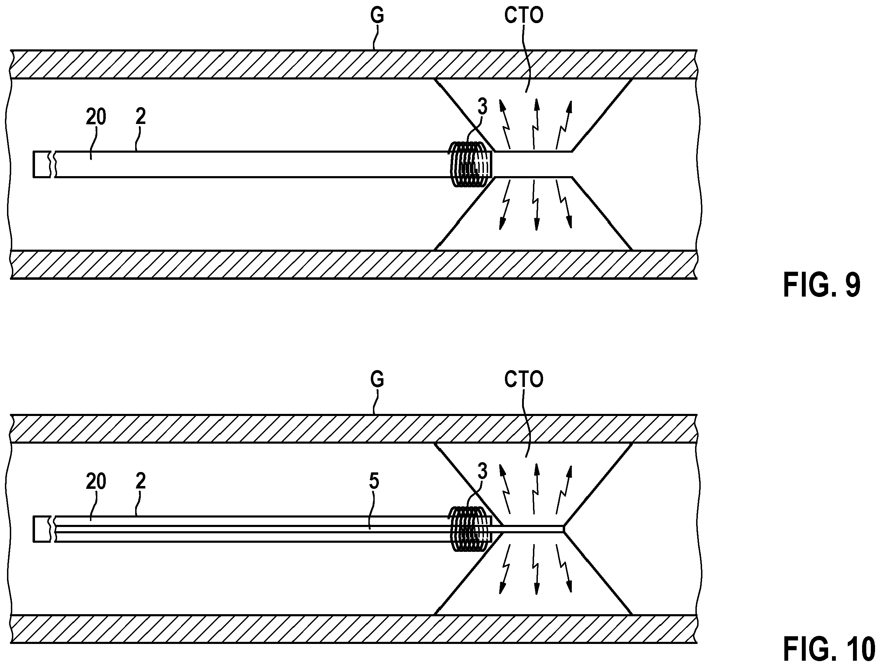

Das Projektil 12 sowie das Federelement 11 werden samt dem längsersteckten Element 10 aus dem Lumen entfernt (vgl. Fig. 9)

Ein Führungsdraht 5 (z.B. ein Standard-Führungsdraht 0.014") wird nun durch das Lumen 20 des Katheterschafts 2 in die geöffnete Okklusion/Stenose CTO eingeführt (vgl. Fig. 10).

Anschließend wird der Katheterschaft 2 aus dem Gefäß G entfernt; der Führungsdraht 5 verbleibt dabei in der geöffneten Okklusion CTO.

Unter Verwendung dieses Führungsdrahts 5 können nun weitere Maßnahmen (z.B. Angioplastie) durchgeführt werden.

Die vorliegende Erfindung ermöglicht es Kardiologen effizienter zu arbeiten. Damit wird das Risiko sowohl für den Anwender als auch für den Patienten reduziert. Eine etwaige Strahlungsbelastung durch Bildgebung beim Eingriff wird mit Vorteil verringert.

## Patentansprüche

1. Kathetereinrichtung (1) mit:
- einem entlang einer Längsachse (x) erstreckten Katheterschaft (2), der einen distalen Endabschnitt (2a) aufweist,
**dadurch gekennzeichnet,**
**dass** die Kathetereinrichtung (1) zum Öffnen der Okklusion (CTO) ein ferromagnetisches Projektil (12) aufweist sowie eine am distalen Endabschnitt (2a) des Katheterschafts (2) angeordnete elektrische Spule (3) zum Beschleunigen des Projektils (12).

2. Kathetereinrichtung nach Anspruch 1, **dadurch gekennzeichnet, dass** der Katheterschaft (2) ein Lumen (20) aufweist.

3. Kathetereinrichtung nach Anspruch 2, **dadurch gekennzeichnet, dass** die elektrische Spule (3) das Lumen (20) in einer Umfangsrichtung (U) des Katheterschafts (2) umgibt.

4. Kathetereinrichtung nach Anspruch 2 oder 3, **dadurch gekennzeichnet, dass** das Projektil (12) über ein Federelement (11) mit einem in dem Lumen (20) angeordneten oder mit einem in das Lumen (20) einführbaren längserstreckten Element (10) verbunden ist.

5. Kathetereinrichtung nach Anspruch 4, **dadurch gekennzeichnet, dass** das längserstreckte Element (10) dazu ausgebildet ist, an der Kathetereinrichtung (1) festgelegt zu werden, insbesondere an einem proximalen Endabschnitt (2b) des Katheterschafts (2), um eine Bewegung des Projektils in distaler Richtung (D) zu begrenzen.

6. Kathetereinrichtung nach Anspruch 4 oder 5, **dadurch gekennzeichnet, dass** das längserstreckte Element (10) durch einen Metalldraht gebildet ist.

7. Kathetereinrichtung nach einem der Ansprüch 4 bis 6, **dadurch gekennzeichnet, dass** das Federelement (11) aus einer diamagnetischen Stahllegierung besteht und/oder dass das längserstreckte Element (10) aus einer diamagnetischen Stahllegierung besteht.

8. Kathetereinrichtung nach einem der vorhergehenden Ansprüche, **dadurch gekennzeichnet, dass** das Projektil (12) aus einer Anfangsposition, in der das Projektil (12) in einem distalen Endabschnitt (20a) des Lumens (20) angeordnet ist, durch Erzeugen eines Magnetfeldes mittels der elektrischen Spule (3) in eine Endposition beschleunigbar ist, in der das Projektil (12) durch eine distale Öffnung (2c) des Katheterschafts (2) aus dem Katheterschaft (2) heraussteht.

9. Kathetereinrichtung nach einem der Ansprüche 4 bis 7 und nach Anspruch 8, **dadurch gekennzeichnet, dass** das Federelement (11) dazu konfiguriert ist, durch Bewegen des Projektils (12) in die Endposition gespannt zu werden, so dass das Federelement (12) eine rückstellende Kraft auf das Projektil (12) ausübt, die das Projektil (12) zurück in die Anfangsposition bewegt.

10. Kathetereinrichtung nach Anspruch 8 oder 9, **dadurch gekennzeichnet, dass** die Kathetereinrichtung (1) eine Steuereinheit (13) aufweist, die dazu konfiguriert ist, einen die elektrische Spule (3) durchfließenden Strom so zu steuern, dass das Magnetfeld wiederholt an und ausgeschaltet wird, derart, dass das Projektil (12) wiederholt zwischen der Anfangsposition und der Endposition hin- und her bewegt wird.

11. Kathetereinrichtung nach Anspruch 10, **dadurch gekennzeichnet, dass** die Steuereinheit (13) mittels entlang des Katheterschafts (2) geführten elektrischen Leitern (4) mit der elektrischen Spule (3) verbunden ist.

12. Verfahren zum Öffnen einer Okklusion (CTO) eines Gefäßes (G) unter Verwendung einer Kathetereinrichtung (1) nach einem der vorhergehenden Ansprüche, aufweisend die Schritte:
- Einführen eines Führungsdrahtes (5) in das Gefäß (G),
- Einführen des Katheterschafts (2) in das Gefäß (G), wobei der Führungsdraht (5) im Lumen (20) angeordnet wird und der Katheterschaft (2) entlang des Führungsdrahtes (5) zur Okklusion (CTO) geführt wird,
- Entfernen des Führungsdrahts (5) und Einführen des Projektils (12) in das Lumen (20) zusammen mit dem längserstreckten Element (10) und dem Federelement (11), und
- wiederholtes Beschleunigen des Projektils (12) gegen die Okklusion (CTO) zum Öffnen der Okklusion (CTO).

13. Verfahren nach Anspruch 12, aufweisend die weiteren Schritte:
- Entfernen des längserstreckten Elementes (10), des Federelements (11) und des Projektils (12) aus dem Lumen (20) des Katheterschafts (2),
- Einführen eines Führungsdrahts (5) in die geöffnete Okklusion (CTO) über das Lumen (20) des Katheterschafts (2), und
- Entfernen des Katheterschafts (2) aus dem Gefäß (G).
